# EUROPEAN PATENT SPECIFICATION

(11) **EP 2 646 011 B1**
(45) Date of publication and mention of the grant of the patent: **16.08.2017**
(21) Application number: 11844637.6
(22) Date of filing: 02.12.2011
(51) Int. Cl.: A61K 31/485, A61K 31/137, A61P 3/04

(54) **METHODS FOR REDUCING BINGE OR COMPULSIVE EATING**
VERFAHREN ZUR REDUZIERUNG VON TRUNK- ODER ESSSUCHT
PROCÉDÉS DE DIMINUTION DE LA FRÉNÉSIE ALIMENTAIRE OU DE COMPORTEMENT ALIMENTAIRE COMPULSIF

(30) Priority: 03.12.2010 US 419354 P
(43) Date of publication of application: 09.10.2013
(73) Proprietor: Orexigen Therapeutics, Inc., La Jolla, CA 92037 (US)
(72) Inventor: DUNAYEVICH, Eduardo, Westlake Village, CA 91362 (US); MCELROY, Susan, Mason, OH 45040 (US); LANDBLOOM, Ron, Jersey City, NJ 07302 (US)
(74) Representative: Zacco Denmark A/S
(86) International application number: PCT/US2011/063170
(87) International publication number: WO 2012/075453

(56) References cited:
- US-A1- 2007 099 947
- US-A1- 2007 129 283
- JONAS J M ET AL: "TREATMENT OF BINGE-EATING AN OPEN-STUDY OF NALTREXONE", SOCIETY FOR NEUROSCIENCE ABSTRACTS, vol. 12, no. 1, 1986, page 595, XP009176761, & 16TH ANNUAL MEETING OF THE SOCIETY FOR NEUROSCIENCE, PART 1, WASHINGTON, D.C., USA, NOV. 9-14, 1986. ISSN: 0190-5295
- MARRAZZI M A ET AL: "Binge eating disorder: response to naltrexone.", INTERNATIONAL JOURNAL OF OBESITY AND RELATED METABOLIC DISORDERS : JOURNAL OF THE INTERNATIONAL ASSOCIATION FOR THE STUDY OF OBESITY FEB 1995, vol. 19, no. 2, February 1995 (1995-02), pages 143-145, XP009176759, ISSN: 0307-0565
- CAMPANA M ET AL: "P.6.034 Naltrexone and craving: Does it work with eating disorders?", EUROPEAN NEUROPSYCHOPHARMACOLOGY, ELSEVIER SCIENCE PUBLISHERS BV, AMSTERDAM, NL, vol. 15, January 2005 (2005-01), page S283, XP027809533, ISSN: 0924-977X [retrieved on 2005-01-01]
- ALGER S A ET AL: "Effect of a tricyclic antidepressant and opiate antagonist on binge-eating behavior in normoweight bulimic and obese, binge-eating subjects.", THE AMERICAN JOURNAL OF CLINICAL NUTRITION APR 1991, vol. 53, no. 4, April 1991 (1991-04), pages 865-871, XP055104254, ISSN: 0002-9165
- NEUMEISTER A ET AL: "Addition of naltrexone to fluoxetine in the treatment of binge eating disorder.", THE AMERICAN JOURNAL OF PSYCHIATRY MAY 1999, vol. 156, no. 5, May 1999 (1999-05), page 797, XP002721418, ISSN: 0002-953X
- HORNE R L ET AL: "Treatment of bulimia with bupropion: a multicenter controlled trial.", THE JOURNAL OF CLINICAL PSYCHIATRY JUL 1988, vol. 49, no. 7, July 1988 (1988-07), pages 262-266, XP009176791, ISSN: 0160-6689
- CARTER WILLIAM P ET AL: "Pharmacologic treatment of binge eating disorder.", THE INTERNATIONAL JOURNAL OF EATING DISORDERS 2003, vol. 34 Suppl, 2003, pages S74-S88, XP055104281, ISSN: 0276-3478
- KENNETT G A ET AL: "New approaches to the pharmacological treatment of obesity: Can they break through the efficacy barrier?", PHARMACOLOGY BIOCHEMISTRY AND BEHAVIOR, ELSEVIER, US, vol. 97, no. 1, November 2010 (2010-11), pages 63-83, XP027453209, ISSN: 0091-3057, DOI: 10.1016/J.PBB.2010.07.020 [retrieved on 2010-08-03]
- HAUSENLOY D J: "Contrave(TM): Novel treatment for obesity", FUTURE LIPIDOLOGY 2009 FUTURE MEDICINE LTD. GBR, vol. 4, no. 3, 2009, pages 279-285, XP009176559, ISSN: 1746-0875
- KRISTELLER ET AL.: 'An Exploratory Study of a Meditation-Based Intervention for Binge Eating Disorder' J HEALTH PSYCHOL vol. 4, no. 3, 1999, pages 357 - 363, XP055100377
- HALPERN ET AL.: 'Combinations of Drugs in the Treatment of Obesity' PHARMACEUTICALS vol. 3, 27 July 2010, pages 2398 - 2415, XP008167210

## Description

### BACKGROUND

### Field of the Disclosure

The present disclosure relates to a composition for use in reducing binge or compulsive eating.

### Description of the Related Technology

Obesity has been defined in terms of body mass index ("BMI"). BMI is calculated as weight (kg)/[height (m)]². According to the guidelines of the U.S. Centers for Disease Control and Prevention ("CDC") and the World Health Organization ("WHO"), for adults over 20 years old, BMI falls into one of the following categories: below 18.5 kg/m² is considered underweight, 18.5-24.9 kg/m² is considered normal, 25.0-29.9 kg/m² is considered overweight, and 30.0 kg/m² and above is considered obese (World Health Organization. Physical status: The use and interpretation of anthropometry. Geneva, Switzerland: World Health Organization 1995. WHO Technical Report Series).

Eating disorders include conditions which may be characterized by abnormal eating habits. The abnormal eating habits may include either insufficient or excessive food intake to the detriment of physical and/or emotional health. Such eating disorders may include, for example, binge eating disorder, bulimia nervosa or anorexia nervosa. Although many eating disorders are associated with women, eating disorders are not gender specific.

Similar to other eating disorders, binge eating disorder is not gender specific. Nevertheless, it is approximately twice as common among women as among men. Further, binge eating disorder is found in all ethno-cultural and racial populations. It was first described in 1959 by psychiatrist and researcher Albert Stunkard as "Night Eating Syndrome" (NES). However, "Binge Eating Disorder" describes binging-type eating behavior without regard to when the behavior occurs. If the person is not already overweight at the time the binge eating behavior starts to manifest itself, the binge eating may lead to the person being overweight or obese.

Binge eating disorder appears common (>20% prevalence) in obese women. Obese women with binge eating disorder exhibit higher anxiety, depression, perceived stress and emotional and external eating scores than obese women without binge eating disorder. High levels of emotional eating and perceived stress can be used to predict binge eating disorder (Pinaquy et al., Obesity Research 2003). Although distinct from binge eating disorder, bulimia nervosa is also commonly associated with depressive symptoms and increased prevalence of depression.

Higher frequency of emotional eating (but not necessarily binge eating disorder) has also been associated with higher baseline BMI. Further, subjects who experience a decrease in emotional eating from an initially high level have been observed to experience greater weight loss than subjects who continued to report high levels of emotional eating (Blair et al., Appetite 1990).

Depression has also been linked to both emotional eating and obesity. For example, atypical depression is often associated with carbohydrate craving and weight gain. Higher BMI has also been associated with negative emotional states in the Diabetes Prevention Program. For example, higher BMI were found to correlate with feeling deprived, angry, or upset while dieting (Delahanty et al., Diabetes Care 2002).

Depression is typically diagnosed as a major depressive disorder (for example, unipolar major depression), dysthymic disorder (for example, dysthymia), and bipolar disorder (for example, manic-depressive illness). There are a number of subtypes of these major categories of depression. For example, atypical depression is a subtype of all three major types of depression. Atypical depression is characterized by the capacity to be cheered up when presented with positive events. Diagnosis of any type of depression or mental disorder may be based on the Diagnostic and Statistical Manual of Mental Disorders, fourth edition (DSM-IV) (American Psychiatric Association; Diagnostic and Statistical Manual of Mental Disorders, fourth edition (DSM-IV), Washington, D.C., American Psychiatric Press, 1994). Major depressive disorder is associated with depressed mood, low energy and motivation, insomnia, and feelings of worthlessness and hopelessness. Dysthymic disorder is considered a milder form of depression with symptoms similar to, but less severe than, those of major depressive disorder. Bipolar disorder is characterized by extreme swings in mood between mania and depression, with mania being accompanied by euphoria, grandiosity, increased energy, decreased need for sleep, rapid speech and risk taking. Exemplary art in the field is Jonas, J. M., et al., "Treatment of binge-eating an open-study of naltrexone", Society for Neuroscience Abstracts, & 16th annual meeting of the society for neuroscience, part 1, washington, d.c., usa, nov. 9-14, 1986., (1986), vol. 12, no. 1, ISSN 0190-5295, page 595, which relates to eating disorders and treatment thereof by administration of naltrexone. Marrazzi, M. A et al, "Binge eating disorder: response to naltrexone.", International Journal of Obesity and Related Metabolic Disorders : Journal of the International Association for the Study of Obesity, Feb 1995, (199502), vol. 19, no. 2, ISSN 0307-0565, pages 143 - 145, which relates to the use of naltrexone to attenuate bulimia nervosa in controlled clinical trials. Campana, M. et al, "P.6.034 Naltrexone and Craving: Does it work with eating disorders?", European Neuropsychopharmacology, Elsevier Science Publishers BV, Amsterdam, NL, vol. 15, ISSN 0924-977x, (200501), page s283, (20050101), which relates to evaluation of efficacy of naltrexone in a group of patients diagnosed with DSM IVtr eating disorders. Alger, S. A. et al, "Effect of a Tricyclic Antidepressant and Opiate Antagonist on Binge-Eating Behavior in Normoweight Bulimic and Obese, Binge-Eating Subjects.", The American Journal of Clinical Nutrition Apr 1991, (199104), vol. 53, no. 4, ISSN 0002-9165, pages 865 - 871, which relates to an 8-week placebo-controlled, double-blind trial investigating the effect of naltrexone and imipramine on 33 obese bingers and 22 bulimics.

### SUMMARY OF CERTAIN INVENTIVE ASPECTS

An embodiment of the invention includes a pharmaceutical composition comprising bupropion or a pharmaceutically acceptable salt thereof, and naltrexone or a pharmaceutically acceptable salt thereof for use in the treatment of binge or compulsive eating.

In some embodiments, the patient is suffering from or at risk of binge eating disorder. In some embodiments, the patient is suffering from or at risk of compulsive eating disorder. In some embodiments, the patient's body mass index is greater than or equal to 25 kg/m2. In some embodiments, the patient's body mass index is greater than or equal to 27 kg/m2. In some embodiments, the patient's body mass index is greater than or equal to 30 kg/m2.

In some embodiments, the patient is not suffering from bipolar disorder.

In some embodiments, the therapeutically effective amount is administered at least once a day. In some embodiments, the therapeutically effective amount is administered for a period of at least 16, 24, 28 or 56 weeks. In some embodiments, the naltrexone or pharmaceutically acceptable salt thereof is administered concurrently with the bupropion or pharmaceutically acceptable salt thereof. In some embodiments, a single oral dosage form comprising naltrexone or pharmaceutically acceptable salt thereof and bupropion or pharmaceutically acceptable salt thereof is administered to the patient. In some embodiments, the therapeutically effective amount of naltrexone or pharmaceutically acceptable salt thereof is 4 mg to 50 mg per day and the therapeutically effective amount of bupropion or pharmaceutically acceptable salt thereof is 30 mg to 500 mg per day. In some embodiments, the therapeutically effective amount of naltrexone or pharmaceutically acceptable salt thereof is 4 mg to 32 mg per day. In some embodiments, the therapeutically effective amount of bupropion or pharmaceutically acceptable salt thereof is 90 mg to 360 mg per day. In some embodiments, the therapeutically effective amount of bupropion or pharmaceutically acceptable salt thereof is 360 mg per day. In some embodiments, the therapeutically effective amount of naltrexone or pharmaceutically acceptable salt thereof is 32 mg per day. In some embodiments, the bupropion comprises a sustained release formulation. In some embodiments, the naltrexone comprises a sustained release formulation. In some embodiments, the patient is female.

In some embodiments, a symptom or measure of the binge eating disorder or the compulsive eating disorder is reduced by at least 5%. In some embodiments, the reduced symptom or measure is strength of binge eating or compulsive eating events. In some embodiments, the reduced symptom or measure is frequency of binge eating or compulsive eating events. In some embodiments, the reduced symptom or measure is the number of binge eating or compulsive eating events. In some embodiments, the patient's binge or compulsive eating is measured using a Binge Eating Scale prior to the start of treatment, and at least once after starting treatment. In some embodiments, the Binge Eating Scale prior to the start of treatment is reduced by at least 10 following treatment. In some embodiments, the Binge Eating Scale is reduced to less than 17 following treatment. In some embodiments, the patient is identified as a patient suffering from or at risk of binge eating disorder or compulsive eating disorder by administration of a Binge Eating Scale checklist.

An embodiment of the invention includes a pharmaceutical composition for use in the treatment of binge or compulsive eating as described in any of the embodiments disclosed herein comprising bupropion or a pharmaceutically acceptable salt thereof and naltrexone or a pharmaceutically acceptable salt thereof.

### DETAILED DESCRIPTION OF CERTAIN INVENTIVE EMBODIMENTS

In general, a person having binge eating disorder is characterized by periodically being unable to exercise control over food consumption. This lack of control leads to eating of an unusually large amount of food at one time, far more than a normal person would eat in the same amount of time. Often the person suffering from binge eating disorder may eat much more quickly during a binge eating episode than during normal eating. The person may eat until he or she is physically uncomfortable and nauseated because of the amount of consumed food. Persons suffering from binge eating disorder may experience rapid weight gain, including, for example, a sudden onset of obesity.

Often the amounts of food consumed are large, even when the person is not hungry. Although the person may not be hungry, the episodes of binge eating may occur when the person is depressed or bored. During a binge eating episode, the binge eater may eat alone so as to avoid discovery or otherwise hide the disorder. Even during episodes of normal eating, however, the binge eater may eat alone due to feelings of embarrassment about food. Following a binge eating episode, the person may experience feelings of disgust, depression or guilt.

Symptoms of binge eating are not merely food cravings, and a reduction in food cravings is not per se a reduction in binge eating. A food craving is an intense desire to consume a specific food, as opposed to general hunger. Certain foods are socially and culturally considered to be "comfort foods," such as, for example, in the United States, ice cream, chocolate, and meat loaf. Individuals who suffer from temporary sadness or depression may crave comfort foods and seek temporary respite from the cause of their unhappiness. It is also well settled that women crave certain foods because of the hormonal changes in their bodies during the normal menstrual cycle or during pregnancy. Food cravings may be common in people following structured diet plans, and such food cravings can be difficult to overcome. Some researchers have suggested that normal food cravings may be caused by the lack of certain nutrients in the body. For example, individuals suffering from lack of iron crave crunchy foods, while hypoglycemic individuals crave pasta or bread, and a person deficient in Vitamin A might crave liver. In contrast, a person having binge eating disorder will periodically be unable to exercise control over food consumption, without regard to the particular food being ingested. Further, unlike food cravings that occur when the person has a hunger, binge eaters will consume large amounts of food without being hungry.

Binge eating symptoms are often present in the eating disorder bulimia nervosa. Bulimia nervosa is an eating disorder characterized by recurrent binge eating, followed by compensatory behaviors. Often these compensatory behaviors include, for example, defensive vomiting (purging), fasting, excessive exercising, or using of laxatives, enemas or diuretics. The formal diagnosis criteria or binge eating disorder and bulimia nervosa are similar in that subjects binge at least twice per week for a minimum period of three months for bulimia nervosa and a minimum of 6 months for binge eating disorder. Unlike in bulimia, however, those with binge eating disorder do not purge, fast or engage in strenuous exercise after binge eating. Additionally, bulimics are typically of normal weight, are underweight but have been overweight before, or are only slightly overweight; persons with binge eating disorder are more likely to be overweight or obese.

Binge eating disorder may also have similar symptoms as compulsive overeating. Compulsive overeating (or food addiction) is characterized by an obsessive/compulsive relationship to food. A person suffering from compulsive overeating disorder engages in frequent episodes of uncontrolled or binge eating, often consuming food past the point of being comfortably full. Similar to binge eating disorder, this binge eating may be followed by feelings of guilt and depression. Compulsive overeaters may also eat when they are not hungry. Unlike persons with bulimia, compulsive overeaters do not attempt to compensate for their binging with purging behaviors such as fasting, laxative use or vomiting. Unlike persons with bulimia or binge eating disorder, however, compulsive overeaters obsess about food. This obsession may be demonstrated in spending excessive amounts of time and thought devoted to food. The obsession may also include secret planning or fantasizing about eating alone. Similar to binge eating disorder, however, compulsive overeating may lead to weight gain and obesity. Although compulsive overeaters tend to be overweight or obese, however, persons of normal or average weight may also be affected. Thus, in contrast to persons suffering from compulsive overeating disorder, persons suffering from binge eating disorder, however, do not have a compulsion to overeat and do not spend a great deal of time fantasizing about food. On the contrary, some people with binge eating disorder have very negative feelings about food.

Binge eating disorder affects approximately four million persons in the United States. Although persons of normal weight may have binge eating disorder, the majority of persons suffering from binge eating disorder are either overweight or obese. Between about 10% and about 15% of persons who are mildly obese also suffer from binge eating disorder. Binge eating disorder is more common, however, in persons who are severely obese.

Because binge eating can manifest itself in many different ways depending on the patient, the Binge Eating Scale ("BES") was developed to assess the severity of binge eating. The BES is a 16-item self-reported checklist designed to assess different levels of binge-eating severity. Gormally, et al., The assessment of binge eating severity among obese persons. Addict Behav. 1982; 7(1) pages 47-55. The BES describes both behavioral manifestations (e.g., eating large amounts of food), and cognitions and feelings surrounding the binge (e.g., guilt or fear of being unable to stop eating). *Gormally* at abstract. The questions are generally aimed at determining the frequency of bingeing, amount of food eaten during a binge, and emotions surrounding a binge. Studies showed that the results of the BES correlated strongly to the opinion of trained observers looking to the frequency of bingeing, amount of food eaten during a binge, and emotions surrounding a binge. *Gormally* at 50-51. Thus, the BES is a reliable tool for determining binge eating severity.

Similar to other eating disorders, binge eating disorder is an "expressive disorder" in that the disorder may be an expression of deeper psychological problems.

### Causes and Complications of Binge Eating Disorder or Binge Eating Episodes

The cause of binge eating disorder is unknown. Some common characteristics may be present in persons having binge eating disorder. For example, about half of all people who have suffered from binge eating disorder have also suffered from depression. Nevertheless, other emotions such as happiness, anger, sadness or boredom may act as trigger points for binge eating episodes. Some people suffering from binge eating disorder claim that binging events occur regardless of mood.

Research also suggests that binge eating disorder may be more common among competitive athletes, such as swimmers or gymnasts, whose body form is (or was) regularly on public display. Some athletes in these sports compare their body in a negative way with those of their teammates.

Binge eating disorder may also be linked to a genetic inheritance factor independent of other obesity risks. Furthermore, there is a higher incidence of psychiatric comorbidity with binge eating disorder.

It is also unclear whether dieting and binge eating are related. Some studies show that about half of all people with binge eating disorder had binge episodes before they started to diet. Furthermore, dieting may be difficult, or even harmful, for those suffering from binge eating disorder. People who are not overweight may make binge eating worse by skipping meals, not eating enough calories per day, or avoiding certain kinds of food such as carbohydrates or fats. Those who are overweight or obese find it difficult to stay in a weight-loss program. They may lose less weight than other people, and regain weight more quickly due to a slowing of the metabolism. Those with additional psychiatric disorders, such as depression, may have an even harder time. Thus, it is often beneficial to treat the binge eating disorder before dieting.

After an episode of binge eating, individuals are often very upset and may become depressed. People tend to overeat from time to time, but a consistent habit of binge eating can lead to weight gain and obesity. Problematic health consequences can occur as a result of the weight gain. Additionally, people may become ill due to a lack of proper nutrition. Bingeing episodes often include foods that are high in fat, sugar, and/or salt, but low in vitamins and minerals. Individuals who binge eat, particularly those who are obese, may also be at risk for type 2 diabetes, high blood pressure (hypertension), high blood cholesterol levels (hypercholesterolemia), gallbladder disease, heart disease, and certain types of cancer.

### Control of Binge Eating Disorder

Many people with binge eating disorder have tried, often unsuccessfully, to control it on their own. Some people miss work, school, or social activities to binge eat. Those who binge eat, whether obese or not, may feel ashamed and attempt to hide the problem. As with other eating disorders, those who suffer from binge eating disorder may become so adept at hiding it that even close friends and family members are unaware that they binge eat.

Several different methods have been used to treat or ameliorate symptoms of binge eating disorder. For example, cognitive-behavioral therapy attempts to teach persons how to keep track of eating and change unhealthy eating habits. It may also teach how to change eating behaviors in tempting or otherwise difficult situations. Interpersonal psychotherapy attempts to teach persons to examine relationships with friends and family and make changes in problem relationships. For persons who are overweight or obese, a weight-loss program in combination with counseling to pinpoint the root of the psychological problems triggering their binge episodes may be used. Other methods may include administration of one or more drugs. These and other methods may be used alone or in combination to effectively treat or ameliorate symptoms of persons suffering from binge eating disorder.

The disclosure of the present invention is directed a pharmaceutical formulation for use in treating or ameliorate symptoms of binge eating disorder. In preferred embodiments, the pharmaceutical formulation comprises a therapeutically effective amount of bupropion or a pharmaceutically acceptable salt thereof, and naltrexone or a pharmaceutically acceptable salt thereof

In certain embodiments, the patient is overweight or obese. In some embodiments, the patient has a body mass index of 25 kg/m² or above. In another aspect of this embodiment, the patient has a body mass index of 27 kg/m² or above. In a further aspect of this embodiment, the patient has a body mass index of 30 kg/m² or above. In certain embodiments, the patient is female.

As discussed, patients who suffer from binge eating disorder may also suffer from a major depressive disorder. However, present invention relates to patients not suffering from bipolar disorder.

In some embodiments, naltrexone and bupropion are each administered once per day to treat binge eating. In some embodiments, naltrexone and bupropion are each divided into equal doses and administered more than once per day. In some embodiments, naltrexone and bupropion are each divided into unequal doses and administered more than once per day. In some embodiments, naltrexone and bupropion are divided into a different number of doses and are administered a different number of times per day. In one such embodiment, the dose of one of naltrexone or bupropion is divided, while the dose of the other is not.

In some embodiments, one or both of naltrexone and bupropion is administered one, two, three, four, or more times per day. In some embodiments, one or both of naltrexone and bupropion are administered in a controlled release formulation.

The exact formulation, route of administration, and dosage for naltrexone and bupropion combinations described herein can be chosen by the individual physician in view of the patient's condition. (*See e.g.,* Fingl et al. 1975, in "The Pharmacological Basis of Therapeutics," Ch. 1 p. 1). In some embodiments, the daily dose of naltrexone and bupropion is the same, and in some embodiments, the daily dose is different.

In some embodiments, the daily dose of naltrexone is, or is about, 4 mg to 50 mg, or 4 mg to 32 mg, or 8 mg to 32 mg, or 8 mg to 16 mg. In some embodiments, the daily dose is, or is about, 4 mg, 8 mg, 12 mg, 16 mg, 32 mg, or 48 mg of naltrexone, or a range defined by any two of the preceding values. In some embodiments, the daily dose is administered in a single oral dosage form. The selection of a particular dosage may be based on the weight of the patient. The selection of a particular dosage may be based on the identity, dosage, and/or dosing schedule of another co-administered compound.

In some embodiments, the daily dose of bupropion is, or is about, 30 mg to 500 mg, or 30 mg to 360 mg, or 90 mg to 360 mg. In some embodiments, the daily dose is, or is about, 30 mg, 90 mg, 180 mg, 360 mg, or 450 mg of bupropion, or a range defined by any two of the preceding values. In some embodiments, the daily dose is administered in a single oral dosage form. The selection of a particular dosage may be based on the weight of the patient.

In some embodiments, at least one of naltrexone or bupropion is administered in consistent daily dosages throughout the period of treatment. In some embodiments, at least one of naltrexone or bupropion is administered in varying daily dosages during the period of treatment. In some of these embodiments, the daily dosages comprise increasing daily dosages over time. In some of these embodiments, the daily dosages comprise decreasing daily dosages over time. In some embodiments, a dosage includes 16 mg of sustained release naltrexone and 180 mg of sustained release bupropion administered daily for a second week.

In some embodiments, naltrexone and bupropion are administered individually. In some embodiments, naltrexone and bupropion are administered in a single pharmaceutical composition comprising naltrexone and bupropion. In some embodiments, at least one of naltrexone or bupropion is in a sustained release or controlled release formulation. For example, sustained release forms of naltrexone are described in U.S. Patent Publication No. 2007/0281021. In some embodiments, at least one of naltrexone or bupropion is administered with a physiologically acceptable carrier, diluent, or excipient, or a combination thereof. Examples of naltrexone/bupropion combinations, formulations thereof, and methods of administering them are disclosed in U.S. Patent Nos. 7,375,111 and 7,462,626,. Reference herein to the use or administration of naltrexone/bupropion combinations will be understood to include all modes of administration disclosed or referred to herein, including administration in sustained release forms. Techniques for formulation and administration of the compounds of the instant application may be found in "Remington's Pharmaceutical Sciences," Mack Publishing Co., Easton, PA, 18th edition, 1990.

In some embodiments, naltrexone is administered prior to the bupropion. In some embodiments, naltrexone is administered subsequent to the bupropion. In some embodiments, naltrexone and the bupropion are co-administered. As used herein, co-administration includes administration in a single dosage form, or separate dosage forms that are administered at, or nearly at, the same time.

In some embodiments, the administration of naltrexone and bupropion is continued for a period of, or of about, 4, 12, 16, 20, 24, 36, 48, or 52 weeks, or a range defined by any two of the preceding values.

The compositions described herein may, if desired, be presented in a pack or dispenser device which may contain one or more unit dosage forms containing one or both of the active ingredients. The pack may, for example, comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human administration. Such notice, for example, may be the labeling approved by the U.S. Food and Drug Administration for prescription drugs, or the approved product insert. The product insert can include, for example, information regarding adverse events, dosage, dosing schedules and efficacy. Compositions comprising a compound of the present disclosure formulated in a compatible pharmaceutical carrier may also be prepared, placed in an appropriate container, and labeled for treatment of an indicated condition. Examples of packs and dispensers as well as oral dosage forms are disclosed in U.S. Patent Publication Nos. 2008-0110792 and 2008-0113026.

Instructions and/or information may be present in a variety of forms, including printed information on a suitable medium or substrate (e.g., a piece or pieces of paper on which the information is printed), computer readable medium (e.g., diskette, CD, etc., on which the information has been recorded), or a website address that may be accessed via the internet. Printed information may, for example, be provided on a label associated with a drug product, on the container for a drug product, packaged with a drug product, or separately given to the patient apart from a drug product, or provided in manner that the patient can independently obtain the information (e.g., a website). Printed information may also be provided to a medical caregiver involved in treatment of the patient.

Using the pharmaceutical compositions described herein, patients may exhibit reduced or fewer symptoms of binge eating when compared to using a placebo, or compared to prior to starting treatment. In some embodiments, patients exhibit fewer or less severe binge or compulsive eating events.

A patient's progress may be monitored by any method that allows one to measure the symptoms of binge or compulsive eating. In an embodiment, the patient's progress may be monitored using a control of eating scale. In certain embodiments, the control of eating scale is the Binge Eating Scale (BES). In certain embodiments, patients may exhibit a decreased BES value during or after treatment when compared with before treatment. In certain embodiments, patients may show a significant change in BES score after 1, 2, 3, 4, 5, 6, 7, 8, 9, 10, 12, 12, 13, 14, or more days, 1, 2, 3, 4, 5, 6, 7, 8, 9, 10, 11, 12, 13, 14, 15, 16, 17, 18, 19, 20, 21, 22, 23, 24 or more weeks, 1, 2, 3, 4, 5, 6, 7, 8, 9, 10, 11, 12 or more months of treatment, or a range defined by any two of the preceding values. In some embodiments, the BES score decreases to below the moderate severity level (≥ 17) for binge eating at some time during or after treatment.

In any of the embodiments disclosed herein, the BES score may be determined at the beginning of the treatment period to measure the severity of binge or compulsive eating in each patient or subject. At various other times in the treatment period a BES is administered. The results of each BES for each patient or subject can be compared with one or more previous or subsequent BES score. In some embodiments treatment results in a significant decrease in the severity of binge or compulsive eating in the subject. In some embodiments, treatment results in a decrease in the frequency of binge or compulsive eating events or the severity of a binge or compulsive eating event. In some embodiments, the decrease in BES score as measured against a starting or prior BES score is, is about, is at least, 5, 6, 7, 8, 9, 10, 15, 20, 25, 30, 35, 40, 45, 50, 55, or 60 %, or is a range defined by any of the preceding values. In some embodiments, the BES score of 10, 11, 12, 13, 14, 15 or 16 questions significantly decreases. In some embodiments, the decrease in BES score for 10, 11, 12, 13, 14, 15 or 16 questions as measured against a starting or prior BES score for the same number of questions is, is about, or is at least, 5, 6, 7, 8, 9, 10, 15, 20, 25, 30, 35, 40, 45, 50, 55, or 60 %. In some embodiments, the measured BES score decreases from severe to moderate. In some embodiments, the measured BES score decreases from severe to low. In some embodiments, the measured BES score decreases from moderate to low. In some embodiments, the measured BES score decreases to below the moderate severity level (≥ 17).

Throughout the present disclosure, when a particular compound is mentioned by name, for example, bupropion or naltrexone, it is understood that the scope of the present disclosure encompasses pharmaceutically acceptable salts.

As used herein, "mitigate" or "mitigation" of binge eating includes preventing or decreasing the amount of weight gain associated with binge eating or with the administration of another drug therapy for binge eating. In some embodiments, mitigation is measured relative to the amount of weight gain typically experienced when only one or neither of naltrexone or bupropion is administered. In some embodiments, mitigation is measured relative to the reduction in numbers of binge eating events. In some embodiments, mitigation is measured relative to the reduction in severity of binge eating events.

As used herein, "promotion" of weight loss includes causing weight loss relative to a baseline weight for a least a portion of the period of treatment. This includes an individual that initially gains some weight, but during the course of treatment loses weight relative to a baseline prior to beginning treatment, as well as individuals that regain a portion or all of the weight that is lost by the end of the treatment period. In a preferred embodiment, at the end of the treatment period, the individual has lost weight relative to a baseline. In a preferred embodiment, mitigation of weight gain or promotion of weight loss in a patient administered naltrexone and bupropion is greater than when neither or only one of naltrexone or bupropion is administered, and more preferably an at least additive, or better than additive, or synergistic, effect of administering the two compounds is achieved.

The term "pharmaceutically acceptable salt" refers to a formulation of a compound that does not cause significant irritation to an organism to which it is administered and does not abrogate the biological activity and properties of the compound. Pharmaceutical salts can be obtained by routine experimentation. Examples of pharmaceutically acceptable salts include bupropion hydrochloride, and naltrexone hydrochloride.

The following examples are intended to illustrate the invention.

### EXAMPLE 1

A 24-week study of sustained release naltrexone and sustained release bupropion for minimization of binge eating in subjects with BMI ≥27 and ≤43 kg/m² was performed. The 25 subjects were between the ages of 18 and 65 and had been diagnosed with major depression, but without other serious medical or psychiatric illness. Table 1 details the history of major depression for all enrolled subjects. As used in Table 1, SD = standard deviation, ^{a} Current episode of major depression was first for subject, ^{b} Subjects had multiple episodes of major depression, but total number was unknown, and ^{c} Age at first depression diagnosis, n=24.

**Table 1**

| **Total number of episodes of major depression** | n=25 |
|---|---|
| 01^{a} | 9(36.0%) |
| 02 | 4(16.0%) |
| 03 | 2 (8.0%) |
| 04 | 4 (16.0%) |
| 05 | 2 (8.0%) |
| 06 | 1 (4.0%) |
| 08 | 1 (4.0%) |
| Unknown^{b} | 2 (8.0%) |

| **Age at first depression diagnosis (years)^{c}** | n=24 |
|---|---|
| Mean (Standard Deviation) | 35.29 (16.11) |
| Median | 40.50 |
| Range (minimum, maximum) | 5.00, 56.00 |

| **Features of current episode of major depression** | n=25 |
|---|---|
| Atypical features | 13 (52.0%) |
| Melancholic features | 12 (48.0%) |
| Other features | 0 |

| **Subject has additional psychiatric history** | n=25 |
|---|---|
| Yes | 21 (84.0%) |
| No | 4 (16.0%) |

| **Duration of current episode (months)** | n=25 |
|---|---|
| Mean (Standard Deviation) | 10.20 (13.98) |
| Median | 6.00 |
| Range (minimum, maximum) | 2.00, 72.00 |

All subjects were currently experiencing an episode of major depression at the start of the study. Thus, subjects who reported 1 episode of major depression were currently experiencing their first episode of major depression. Subjects with an unknown number of episodes of major depression reported multiple episodes but the total number was unknown.

The study was conducted for up to 24 weeks. The first four weeks is considered the titration period and the following 20 weeks is considered the maintenance period. During the first week, subjects took one tablet (8 mg of sustained release naltrexone and 90 mg of sustained release bupropion) in the morning. During the second week, subjects took one tablet in the morning and one tablet in the evening. Subjects took two tablets in the morning and one tablet in the evening for the third week. During the fourth week, subjects took two tablets in the morning and two tablets in the evening. The twice daily doses were to be separated by at least 8 hours. During the maintenance period of 20 weeks following the titration period, subjects took two tablets twice daily with food. Additionally, subjects participated in ancillary therapy that included meetings with study staff, diet instruction, advice on diet behavior modification and a prescription for exercise.

Each subject completed a BES checklist at baseline (Day 1) and at Weeks 4, 8, 12, and 24 (or at early termination). Binge Eating Scale (BES) is a 16-item self-reported checklist designed to assess different levels of binge-eating severity. Gormally, et al., The assessment of binge eating severity among obese persons. Addict Behav. 1982; 7(1) pages 47-55. Subjects were instructed to read all statements in each group of the checklist and place a mark next to the statement that best describes their eating behavior. The BES total score is the sum of the 16 items and ranges from 0 to 46. A BES total score of ≥ 17 indicates a degree of severity that is at least moderate.

The 16-item BES assessed different levels of binge eating during the study. At screening, 18 of the 25 enrolled subjects had a history of binge eating disorder. The mean BES score total score for the full analysis set was 28.74 and the median was 31.00 at baseline. At week 12 (LOCF), the mean BES total score was 13.95, with a mean decrease from baseline of 14.67. At week 24 (LOCF), the mean BES total score was 12.57, with a mean decrease from baseline of 16.05. The mean BES scores at both Week 12 and Week 24 (LOCF) decreased from baseline to below the moderate severity level (≥ 17) for binge eating.

Other efficacy measures were: change in waist circumference; serum leptin and ghrelin levels; salivary cortisol levels; safety and tolerability. Adverse events and vital signs (e.g., systolic and diastolic blood pressure and pulse) were used to monitor safety and tolerability. With the exception of the change in waist circumference and salivary cortisol levels, secondary measures indicated change that was generally consistent. The lack of treatment effect on waist circumference and salivary cortisol levels may be due to small sample size or high variability of these measures.

Table 2 details the BES Total Score at baseline, 12 weeks, 24 weeks (LOCF), and the change from baseline at 12 and 24 weeks. As used in Table 2, BES = Binge Eating Scale; CI = confidence interval; LOCF = last observation carried forward; and SD = standard deviation. Endpoint is Week 12, Week 24, or the last non-missing measurement prior to either Week 12 or Week 24 for analysis on Week 12 or Week 24, respectively. T-tests were calculated to evaluate whether the change from baseline was statistically significant from zero, with the 95% CI reported. Results were based on the LOCF method. If <20% of the scale item scores were missing, the scale total score was imputed by multiplying the average of the non-missing scale item scores by the total number of scale item scores possible. If ≥20% of scale item scores were missing, the scale total score was set to missing. Full analysis set included all subjects who received study drug, had a baseline measurement, and had at least one postbaseline MADRS total score measurement while on study drug.

**Table 2**

| **Timepoint** | **BES Total Score** |
|---|---|
| **Baseline** | n=23 |
| Mean (Standard Deviation) | 28.74 (8.70) |
| Median (range) | 31.00 (8.00 to 41.00) |
| 95% Confidence Interval (lower, upper) | (24.98, 32.50) |

| **Week 12** | n=21 |
|---|---|
| Mean (Standard Deviation) | 13.95 (10.62) |
| Median (range) | 16.00 (0.00 to 35.00) |
| 95% Confidence Interval (lower, upper) | (9.12, 18.79) |

| **Week 12: Change from Baseline** | n=21 |
|---|---|
| Mean (Standard Deviation) | -14.67 (9.98) |
| Median (range) | -12.00 (-35.00 to -3.00) |
| 95% Confidence Interval (lower, upper) | (-19.21, -10.12) |
| p-value | <0.001 |

| **Week 24** | n=21 |
|---|---|
| Mean (Standard Deviation) | 12.57 (10.83) |
| Median (range) | 9.00 (0.00 to 35.00) |
| 95% Confidence Interval (lower, upper) | (7.64, 17.50) |

| **Week 24: Change from Baseline** | n=21 |
|---|---|
| Mean (Standard Deviation) | -16.05 (9.62) |
| Median (range) | -16.00 (-35.00 to -2.00) |
| 95% Confidence Interval (lower, upper) | (-20.43, -11.67) |
| p-value | <0.001 |

There were a number of changes to BES responses for each of the 16 questions over the 24 week period. Responses to each of the BES questions indicated binge eating was mitigated by administration of naltrexone and bupropion over the course of the titration period and the maintenance period.

### EXAMPLE 2

A study of sustained release naltrexone and sustained release bupropion for treatment of binge or compulsive eating is provided. Subjects are identified as experiencing binge or compulsive eating based upon a Binge Eating Scale ("BES") checklist or an equivalent diagnostic measure (e.g., professional assessment). Binge or compulsive eating severity is measured by the severity of individual events and/or by the frequency of such events. Subjects are also identified as having an initial body mass index ("BMI") ≥30 kg/m². Subjects are not limited based upon race or gender.

Subjects are provided with an oral dosage of 8 mg sustained release naltrexone and 90 mg sustained release bupropion a day for 1 week, 16 mg sustained release naltrexone and 180 mg sustained release bupropion a day for a second week, 24 mg sustained release naltrexone and 270 mg sustained release bupropion a day for a third week, and 32 mg sustained release naltrexone and 360 mg sustained release bupropion a day thereafter for the course of the study.

A baseline BES score or equivalent is obtained before treatment begins, and at least once more after at least one week of treatment. Subjects show a significant reduction in one or more measures of binge and/or compulsive eating as measured by the BES or equivalent following treatment as compared to baseline and/or one or more prior BES scores or equivalent during treatment.

### EXAMPLE 3

In another study, subjects are identified in the same manner as Example 2. Subjects are divided into two groups. Group 1 is provided with an oral dose of sustained release naltrexone and an oral dosage of sustained release bupropion in the same manner and over the same treatment period as described in Example 2. Group 2 is administered placebo in the same manner as the oral dosages that are provided to Group 1. Both Group 1 and Group 2 participate in the study for the same treatment period.

A baseline BES score or equivalent is obtained before treatment begins, and at least once more after at least one week of treatment. Subjects receiving naltrexone/bupropion treatment show a significant reduction in one or more measures of binge and/or compulsive eating as measured by a BES or equivalent as compared to baseline and/or one or more prior BES scores or equivalent during treatment, as well as in comparison to the placebo treatment group at baseline and/or at one or more time points during treatment.

### EXAMPLE 4

In another study, subjects are identified as experiencing binge or compulsive eating based upon a BES checklist or equivalent as described in Example 2. Subjects are also identified as having an initial BMI ≥27 kg/m² and having one or more risk factors, including diabetes, dyslipidemia, or hypertension. Subjects are not limited based upon race or gender. Subjects are provided with an oral dosage form of sustained release naltrexone and an oral dosage of sustained release bupropion in the same dosage in the same manner for the same treatment period as described above in Example 2.

A baseline BES score or equivalent is obtained before treatment begins, and at least once more after at least one week of treatment. Subjects show a significant reduction in one or more measures of binge and/or compulsive eating as measured by the BES or equivalent following treatment as compared to baseline and/or one or more prior BES scores or equivalent during treatment.

### EXAMPLE 5

In another study, subjects are identified in the same manner as Example 4. Subjects are divided into two groups. Group 1 is provided with an oral dose of sustained release naltrexone and an oral dosage of sustained release bupropion in the same manner and for the same treatment period as described in Example 2. Group 2 is provided with placebo for administration for the same treatment period in the same manner as the oral dosages provided in Group 1. Both Group 1 and Group 2 participate in the second study for the same treatment period.

A baseline BES score or equivalent is obtained before treatment begins, and at least once more after at least one week of treatment. Subjects receiving naltrexone/bupropion treatment show a significant reduction in one or more measures of binge and/or compulsive eating as measured by a BES or equivalent as compared to baseline and/or one or more prior BES scores or equivalent during treatment, as well as in comparison to the placebo treatment group at baseline and/or at one or more time points during treatment.

### EXAMPLE 6

In another study, subjects are identified as experiencing binge or compulsive eating based upon a BES checklist or equivalent as described in Example 2. Subjects are also identified as having an initial BMI ≥27 kg/m². Subjects are not limited based upon race or gender. Subjects are divided into two groups. Group 1 includes subjects suffering from depression or a depression related disorder. Group 2 includes subjects without depression or a depression related disorder. Subjects in both Group 1 and Group 2 are provided with an oral dosage form of sustained release naltrexone and an oral dosage of sustained release bupropion in the same dosage in the same manner for the same treatment period as described above in Example 2.

A baseline BES score or equivalent is obtained before treatment begins, and at least once more after at least one week of treatment. Subjects in both Group 1 and Group 2 show a significant reduction in one or more measures of binge and/or compulsive eating as measured by the BES or equivalent following treatment as compared to baseline and/or one or more prior BES scores or equivalent during treatment.

## Claims

1. A pharmaceutical composition comprising bupropion or a pharmaceutically acceptable salt thereof, and naltrexone or a pharmaceutically acceptable salt thereof for use in the treatment of binge or compulsive eating.

2. The pharmaceutical composition for use according to claim 1, further comprising: instructing the patient to daily administer the therapeutically effective amount.

3. The pharmaceutical composition for use according to any of Claims 1-2, wherein the patient's body mass index is greater than or equal to 25 kg/m².

4. The pharmaceutical composition for use according to any of Claims 1-3, wherein the patient is not suffering from a major depressive disorder.

5. The pharmaceutical composition for use according to any of Claims 1-4 further comprising identifying the patient as suffering from a major depressive disorder.

6. The pharmaceutical composition for use according to Claim 5, wherein the patient is not suffering from bipolar disorder.

7. The pharmaceutical composition for use according to any of Claims 1-6, wherein the therapeutically effective amount of naltrexone or pharmaceutically acceptable salt thereof is 4 mg to 50 mg per day and the therapeutically effective amount of bupropion or pharmaceutically acceptable salt thereof is 30 mg to 500 mg per day.

8. The pharmaceutical composition for use according to any of Claims 1-7, wherein the patient is female.

9. The pharmaceutical composition for use according to any of Claims 1-8, wherein a symptom or measure of the binge eating disorder or the compulsive eating disorder is reduced by at least 5%.

10. The pharmaceutical composition for use according to Claim 9, wherein the reduced symptom or measure is strength of binge eating or compulsive eating events.

11. The pharmaceutical composition for use according to Claim 9, wherein the reduced symptom or measure is frequency of binge eating or compulsive eating events.

12. The pharmaceutical composition for use according to any of Claims 1-11, wherein the patient's binge or compulsive eating is measured using a Binge Eating Scale prior to the start of treatment, and at least once after starting treatment.

13. The pharmaceutical composition for use according to Claim 12, wherein the value of the Binge Eating Scale prior to the start of treatment is reduced by at least 10 following treatment or is reduced to less than 17 following treatment.

14. The pharmaceutical composition for use according to any of Claims 1-13, wherein the patient is identified as a patient suffering from or at risk of binge eating disorder or compulsive eating disorder by administration of a Binge Eating Scale checklist.

15. The pharmaceutical composition for use according to any of claims 1 to 14 further comprising:
identifying a patient suffering from or at risk of suffering from binge or compulsive eating by administration of a binge eating scale checklist, the patient having a body mass index (BMI) greater than or equal to 27 kg/m2; and
administering to the patient 16 mg sustained release naltrexone and 180 mg sustained release bupropion twice daily for a treatment period of more than 4 weeks.

## Patentansprüche

1. Pharmazeutische Zusammensetzung umfassend Bupropion oder ein pharmazeutisch verträgliches Salz davon und Naltrexon oder ein pharmazeutisch verträgliches Salz davon zur Anwendung bei der Behandlung von Binge Eating oder zwanghaftem Essen.

2. Pharmazeutische Zusammensetzung zur Anwendung nach Anspruch 1, zusätzlich umfassend: der Patient wird angewiesen, die therapeutisch wirksame Menge täglich einzunehmen.

3. Pharmazeutische Zusammensetzung zur Anwendung nach einem der Ansprüche 1 bis 2, wobei die Körpermassenzahl des Patienten größer als oder gleich 25 kg/m² ist.

4. Pharmazeutische Zusammensetzung zur Anwendung nach einem der Ansprüche 1 bis 3, wobei der Patient nicht unter einer schweren depressiven Störung leidet.

5. Pharmazeutische Zusammensetzung zur Anwendung nach einem der Ansprüche 1 bis 4, zusätzlich umfassend die Identifizierung des unter einer schweren depressiven Störung leidenden Patienten.

6. Pharmazeutische Zusammensetzung zur Anwendung nach Anspruch 5, wobei der Patient nicht unter einer bipolaren Störung leidet.

7. Pharmazeutische Zusammensetzung zur Anwendung nach einem der Ansprüche 1 bis 6, wobei die therapeutisch wirksame Menge von Naltrexon oder einem pharmazeutisch verträglichen Salz davon 4 mg bis 50 mg pro Tag beträgt, und die therapeutisch wirksame Menge von Bupropion oder einem pharmazeutisch verträglichen Salz davon 30 mg bis 500 mg pro Tag beträgt.

8. Pharmazeutische Zusammensetzung zur Anwendung nach einem der Ansprüche 1 bis 7, wobei der Patient weiblich ist.

9. Pharmazeutische Zusammensetzung zur Anwendung nach einem der Ansprüche 1 bis 8, wobei ein Symptom oder Ausmaß der Binge-Eating-Störung oder der zwanghaften Essstörung um mindestens 5 % reduziert wird.

10. Pharmazeutische Zusammensetzung zur Anwendung nach Anspruch 9, wobei das reduzierte Symptom oder Ausmaß die Intensität von Ereignissen von Binge-Eating oder zwanghaftem Essen ist.

11. Pharmazeutische Zusammensetzung zur Anwendung nach Anspruch 9, wobei das reduzierte Symptom oder Ausmaß die Häufigkeit von Ereignissen von Binge-Eating oder zwanghaftem Essen ist.

12. Pharmazeutische Zusammensetzung zur Anwendung nach einem der Ansprüche 1 bis 11, wobei das Bing Eating oder das zwanghafte Essen des Patienten unter Anwendung einer Binge-Eating-Scale vor dem Beginn der Behandlung und zumindest einmal nach dem Behandlungsbeginn gemessen wird.

13. Pharmazeutische Zusammensetzung zur Anwendung nach Anspruch 12, wobei der Wert der Binge-Eating-Scale vor dem Beginn der Behandlung um zumindest 10 nach der Behandlung oder auf weniger als 17 nach der Behandlung reduziert wird.

14. Pharmazeutische Zusammensetzung zur Anwendung nach einem der Ansprüche 1 bis 13, wobei der Patient als ein Patient, der unter einer Binge-Eating-Störung oder einer zwanghaften Essstörung leidet oder gefährdet ist, darunter zu leiden, durch die Verwendung einer Binge-Eating-Scale-Checkliste identifiziert wird.

15. Pharmazeutische Zusammensetzung zur Anwendung nach einem der Ansprüche 1 bis 14, zusätzlich umfassend:
Identifizierung eines Patienten, der unter Bing Eating oder zwanghaftem Essen leidet oder gefährdet ist, darunter zu leiden, durch die Verwendung einer Binge-Eating-Scale-Checkliste, wobei der Patient eine Körpermassenzahl (BMI) aufweist, die größer als oder gleich 27 kg/m2 ist; und
Verabreichung von 16 mg nachhaltiger Freisetzung von Naltrexon und 180 mg nachhaltiger Freisetzung von Bupropion an den Patienten zweimal täglich für einen Behandlungszeitraum von mehr als 4 Wochen.

## Revendications

1. Composition pharmaceutique comprenant le bupropion ou l'un de ses sels pharmaceutiquement acceptables, et la naltrexone ou l'un de ses sels pharmaceutiquement acceptables pour l'usage dans le traitement de la frénésie alimentaire ou du comportement alimentaire compulsif.

2. Composition pharmaceutique pour l'usage selon la revendication 1, comprenant en outre: l'instruction au patient d'administrer quotidiennement la quantité thérapeutiquement efficace.

3. Composition pharmaceutique pour l'usage selon l'une quelconque des revendications 1 à 2, dans laquelle l'indice de masse corporelle du patient est supérieur ou égal à 25 kg/m².

4. Composition pharmaceutique pour l'usage selon l'une quelconque des revendications 1 à 3, dans laquelle le patient ne souffre pas d'un trouble dépressif majeur.

5. Composition pharmaceutique pour l'usage selon l'une quelconque des revendications 1 à 4, comprenant en outre l'identification du patient comme souffrant d'un trouble dépressif majeur.

6. Composition pharmaceutique pour l'usage selon la revendication 5, dans laquelle le patient ne souffre pas d'un trouble bipolaire.

7. Composition pharmaceutique pour l'usage selon l'une quelconque des revendications 1 à 6, dans laquelle la quantité thérapeutiquement efficace de naltrexone ou de son sel pharmaceutiquement acceptable est comprise entre 4 mg et 50 mg par jour, et la quantité thérapeutiquement efficace de bupropion ou son sel pharmaceutiquement acceptable est comprise entre 30 mg et 500 mg par jour.

8. Composition pharmaceutique pour l'usage selon l'une quelconque des revendications 1 à 7, dans laquelle le patient est une femme.

9. Composition pharmaceutique pour l'usage selon l'une quelconque des revendications 1 à 8, dans laquelle un symptôme ou une mesure de la frénésie ou du comportement alimentaire compulsif est réduit d'au moins 5%.

10. Composition pharmaceutique pour l'usage selon la revendication 9, dans laquelle le symptôme réduit ou la mesure réduite est la force des événements de la frénésie alimentaire ou du comportement alimentaire compulsif.

11. Composition pharmaceutique pour l'usage selon la revendication 9, dans laquelle le symptôme réduit ou la mesure réduite est la fréquence des événements de la frénésie alimentaire ou du comportement alimentaire compulsif.

12. Composition pharmaceutique pour l'usage selon l'une quelconque des revendications 1 à 11, dans laquelle la frénésie alimentaire ou le comportement alimentaire compulsif du patient est mesuré à l'aide d'une échelle Binge Eating Scale avant le début du traitement et au moins une fois après le début du traitement.

13. Composition pharmaceutique pour l'usage selon la revendication 12, dans laquelle la valeur de l'échelle Binge Eating Scale avant le début du traitement est réduite d'au moins 10 suivant le traitement ou est réduite à moins de 17 suivant le traitement.

14. Composition pharmaceutique pour l'usage selon l'une quelconque des revendications 1 à 13, dans laquelle le patient est identifié comme un patient souffrant d'un trouble de la frénésie alimentaire ou d'un trouble du comportement alimentaire compulsif ou à son risque, par l'administration d'une liste de contrôle de l'échelle Binge Eating Scale.

15. Composition pour l'usage selon l'une quelconque des revendications 1 à 14, comprenant en outre:
l'identification d'un patient souffrant ou risquant de souffrir de la frénésie alimentaire ou du comportement alimentaire compulsif par l'administration d'une liste de contrôle de l'échelle Binge Eating Scale, le patient ayant un indice de masse corporelle (IMC) supérieur ou égal à 27 kg/m2; et
l'administration au patient de 16 mg de naltrexone à libération prolongée et de 180 mg de bupropion à libération prolongée deux fois par jour pendant une période de traitement de plus de 4 semaines.
